# EUROPEAN PATENT SPECIFICATION

(11) **EP 0 526 527 B1**
(45) Date of publication and mention of the grant of the patent: **30.07.1997**
(21) Application number: 91908504.3
(22) Date of filing: 09.04.1991
(51) Int. Cl.: A61M 25/01

(54) **HIGH ELONGATION LINEAR ELASTIC GUIDEWIRE**
LINEARER ELASTISCHER FÜHRUNGSDRAHT MIT GROSSER DEHNFÄHIGKEIT
FIL DE GUIDAGE ELASTIQUE DE GRANDE ELONGATION

(30) Priority: 10.04.1990 US 507375
(43) Date of publication of application: 10.02.1993
(73) Proprietor: Boston Scientific Corporation, Natick, Massachusetts 01760 (US)
(72) Inventor: SAHATJIAN, Ronald, Lexington, MA 02173 (US); ALVAREZ DE TOLEDO, Fernando, Concord, MA 01742 (US)
(74) Representative: Kador & Partner
(86) International application number: US9102420
(87) International publication number: WO9115152

(56) References cited:
- WO-A-88/04940
- US-A- 3 351 463
- US-A- 4 925 445
- US-A- 4 991 602

## Description

### Field of the Invention

This invention relates to guidewires for navigating internal passageways of a body.

### Background of the Invention

Generally, the distal end of a guidewire is introduced into a body by a physician, e.g., through a puncture opening. The physician manipulates the tip of the guidewire through tortuous aspects of the body's passageways to a site to be treated. A catheter or other medical device is advanced over the guidewire to the treatment site and the guidewire is then removed, leaving the catheter in place.

In order for the physician to have maximal control over the guidewire, and to ensure the patient's safety, it is important that the guidewire be as small in diameter as possible, particularly in the tip region, but not so small as to create a danger of the tip breaking loose in the body. It is also important that the guidewire be smooth to allow ready advancement and retraction within the passageways; that the distal tip of the guidewire be highly flexible to permit negotiation of difficult turns within the body; that the guidewire be stiff enough axially to be advanced by thrust from the proximal end outside the body without kinking, i.e., turning back upon itself; and that the guidewire have good steerability or torque response. Most prior art guidewires compromise these desired features.

To aid the steering of the element into a desired lumen, it is common to give at least the tip portion of the element a predetermined curvature that complements the lumen path. Many guidewires available today have a curvature formed in the factory during manufacture. Some guidewires made of conventional materials, e.g., stainless steel, can be tip-formed by the physician prior to insertion, a feature found desirable by many.

Fuji Terumo Co. Ltd., EPA 0 141 006 describes a guidewire having at least portions of the body and/or flexible distal end formed of a superelastic metal member, e.g., a specially heat treated Ti-Ni alloy (Nitinol). The end may have a curved tip to aid steering. Because of the high elongation of the superelastic materials, a guidewire which has been previously curvedly deformed can be straightened when being introduced to the body through a needle and then will restore itself to the original curved shape when inserted in a blood vessel.

In WO 88/04940, also by Terumo, a guidewire is formed by differentially heat treating a linear material, e.g., an elastic alloy such as an ultra elastic alloy (e.g., Ni-Ti) so that the flexibility increases progressively from the base to the tip.

### Summary of the Invention

Generally, the invention features a guidewire, preferably an angiographic guidewire and a method for its manufacture. In one aspect, the invention features a guidewire device having a distal portion comprised of a precursor of a superelastic alloy. The distal portion has a stress-strain curve exhibiting a yield point without passing through a substantial plateau. The distal portion of the guidewire is deformable beyond the yield point to a desired set shape.

The invention comprehends a guidewire device having a distal portion comprised of an alloy having the composition of a superelastic alloy without exhibiting a phase transition in response to deforming stress and having a linear stress-strain curve extending to a yield point without passing through a substantial plateau in which stress remains substantially constant while strain is varied, the distal alloy portion of said guidewire being deformable beyond said yield point to a desired set shape: such an alloy may be regarded as a "precursor" of a superelastic alloy and will, for convenience, be from time to time referred to as such herein.

In various aspects of the guidewire device, the precursor is in the martensite phase at room temperature to body temperature; the precursor is selected from the group consisting of an alloy of In-Tl, Fe-Mn, Ni-Ti, Ag-Cd, Au-Cd, Au-Cu, Cu-Al-Ni, Cu-Au-Zn, Cu-Zn, Cu-Zn-Al, Cu-Zn-Sn, Cu-Zn-Xe, Fe₃Be, Fe₃Pt, Ni-Ti-V, Fe-Ni-Ti-Co, and Cu-Sn; preferably the alloy is selected from the group consisting of Ni-Ti, Cu-Al-Ni and Cu-Zn-Al; more preferably, the precursor is an alloy of about 55%/45% Ni-Ti; at least part of the distal portion is smaller than an integral portion of the guidewire proximal thereof which is also comprised of the precursor of a superelastic alloy; at least part of the portion formed of the precursor is tapered; the guidewire includes a core element comprised of the precursor, the core element extending proximally, integrally from the element of the distal portion; the guidewire includes a polymeric coating; the coating is polytetrafluoroethylene; the coating is a lubricious polymer; the coating includes an antithrombogenic agent; the agent is heparin; the distal portion has an element comprised of the precursor of a superelastic alloy and further a flexible spring coil surrounds the element; the spring coil is more flexible than the element; the guidewire may be in the form of an angiographic guidewire having a total length of about 145 cm, or a gastrointestinal (GI) guidewire with a total length of about 450cm, either of which may have a body portion of about 0.5 to 7.4 mm (0.020 to 0.290 inch) diameter, a taper portion about 7 to 10 cm long and a tip portion about 2 to 10 cm long with a diameter of about 0.178 mm (0.007 inch).

In another aspect, the invention features a method of forming a guidewire device by providing an element comprised of a precursor of a superelastic alloy, the element having a linear stress-strain curve exhibiting a yield point, without passing through a substantial plateau and reducing the diameter of at least a portion of the element under conditions avoiding substantial heating. Thereafter, the element is incorporated into a guidewire device, with the reduced diameter portion forming part of the distal portion of the guidewire device. The distal portion is elastically flexible while being capable of being given a desired permanent set by manual stressing of the distal region beyond its yield point.

In various aspects of the method, the element is selected from In-Tl, Fe-Mn, Ni-Ti, Ag-Cd, Au-Cd, Au-Cu, Cu-Al-Ni, Cu-Au-Zn, Cu-Zn, Cu-Zn-Al, Cu-Zn-Sn, Cu-Zn-Xe, Fe₃Be, Fe₃Pt, Ni-Ti-V, Fe-Ni-Ti-Co, and Cu-Sn; preferably the element is 55%/45% Ni-Ti; the method further includes selecting an element in the martensite phase at room temperature to body temperature and maintaining the martensite phase during the formation of the guidewire; the maintaining includes maintaining the temperature of the element below the transition temperature to the austenite phase; the element is maintained at room temperature; the method includes grinding at least part of the element to a reduced diameter while flooding the element with cooling fluid to control the temperature of the element; the method includes controlling the grinding speed to control the temperature of the element; the temperature during grinding is below 300°F; the temperature during grinding is room temperature; the method includes grinding the element to provide a taper; the method includes drawing the element to produce the reduced diameter portion; the method includes chemical etching of the element to produce the reduced diameter portion; the method includes applying to the element, a polymer coating; the method includes applying to the element, a lubricious coating; the method includes applying to the element, an antithrombogenic coating.

In yet another aspect, the invention features a guidewire device comprising, in at least its distal portion, an element consisting of a metal alloy having the elemental composition of a superelastic alloy and at room temperature to body temperature a substantially linear stress strain curve exhibiting a yield point without passing through a substantial plateau. The distal portion of the guidewire is deformable beyond the yield point to a desired set shape.

Preferably, the alloy is selected from In-Tl, Fe-Mn, Ni-Ti, Ag-Cd, Au-Cd, Au-Cu, Cu-Al-Ni, Cu-Au-Zn, Cu-Zn, Cu-Zn-Al, Cu-Zn-Sn, Cu-Zn-Xe, Fe₃Be, Fe₃Pt, Ni-Ti-V, Fe-Ni-Ti-Co, and Cu-Sn.

In another aspect the invention features a method of forming a guidewire device by providing an element comprised of an alloy having the composition of a superelastic alloy and at room to body temperature having a linear stress-strain curve exhibiting a yield point without passing through a substantial plateau, the element having initial properties of elasticity and straightness and reducing the diameter of at least a portion of the guidewire under conditions to maintain the initial properties. Thereafter, the element is incorporated into a guidewire device, with the reduced diameter portion forming part of the distal portion of the guidewire device, the distal portion being elastically flexible while being capable of assuming a desired permanent set by application of manual stressing of the distal region beyond its yield point.

In various aspects, the method includes forming the element by cold drawing to impart the initial properties; the alloy is selected from In-Tl, Fe-Mn, Ni-Ti, Ag-Cd, Au-Cd, Au-Cu, Cu-Al-Ni, Cu-Au-Zn, Cu-Zn, Cu-Zn-Al, Cu-Zn-Sn, Cu-Zn-Xe, Fe₃Be, Fe₃Pt, Ni-Ti-V, Fe-Ni-Ti-Co, and Cu-Sn.

These and other features and advantages will be seen from the following description of a presently preferred embodiment, and from the claims.

### Description of the Preferred Embodiment

We first briefly describe the drawings.

### Drawings

Fig. 1 is a plan view of the core of a guidewire of the invention;
Fig. 2 is a plan view partially cutaway of a guidewire having a core as in Fig. 1 and including a polymer coating;
Fig. 3 is a plan view of a guidewire having a core as in Fig. 1 and including a distal element coil.
Figs. 4 and 4a, illustrate idealized stress-strain curves for superelastic (Fig. 4) and linear elastic (Fig. 4a) materials.
Figs. 5-5c illustrate the use of the guidewire of the invention.
Fig. 6 compares stress strain curves from various cores.
Fig. 7 illustrates the experimental apparatus used to obtain test results as described in Test Example 2.

### Structure

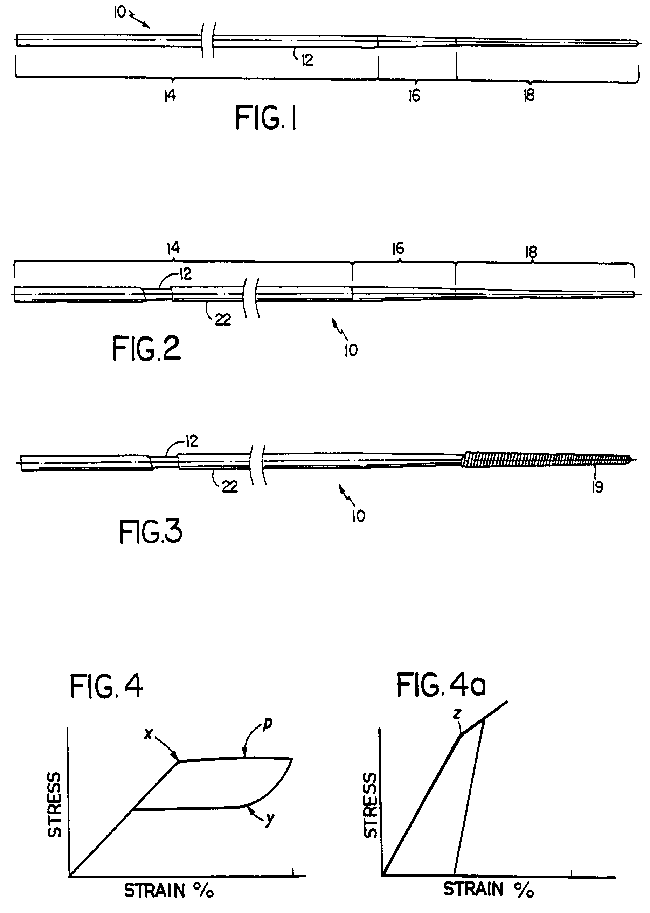

Referring to Figs. 1 to 3, a guidewire 10 of the invention has an elongated core element 12 formed of a precursor of a superelastic alloy. The core 12 includes a proximal body section 14 of constant diameter, a taper 16 and a distal end 18 of constant, smaller diameter than the body section 14.

According to the invention, the core 12 is formed of a linear elastic precursor of a superelastic (or pseudo-elastic) metal alloy. The guidewire core is manufactured from a raw element that is made of the precursor alloy. The raw element is made by repeatedly drawing ingots of the precursor alloy without the application of heat (cold drawing). The alloy precursors of superelastic alloys are in the martensite crystalline phase at room temperature to body temperature and are linear elastic materials, i.e., they are easily permanently bent (plastically deformed) upon reaching a given stress and strain, known as the yield point, without passing through a substantial plateau.

Raw elements formed of stress-induced superelastic materials, on the other hand, are formed by drawing an ingot of the precursor alloy while simultaneously heating. In the unstressed state at room temperature such superelastic materials occur in the austenite crystalline phase and upon application of stress, exhibit stress-induced austenite-martensite crystalline transformations which produce non-linear elastic behavior. As a result, superelastic materials are able to undergo repeated high strain deformations without plastic deformation. When the deforming stress is removed the strain is recovered and there is little or no permanent or plastic deformation.

It is an advantage of superelastic materials that, after imparting the superelastic properties by proper processing, they may be highly strained (deformed), yet still regain their original shape upon release of the deforming stress. It is an advantage of common linear elastic materials that they can be conveniently and easily plastically deformed to a desired shape with relatively little strain.

The present invention contemplates guidewires formed of certain special alloys that are highly elastic, i.e. have high strain prior to yield, although typically not as high as the maximum strain of superelastic materials, but are also easily plastically deformable. Such alloys are those that are precursors of superelastic alloys, i.e., they have the same chemical constituents as superelastic alloys, but have not been processed to impart the superelastic property.

Referring to Figs. 4 and 4a, schematic stress versus strain curves for a superelastic alloy (Fig. 4) and a super elastic precursor alloy that exhibits linear elastic properties (Fig. 4a) are compared. For a superelastic alloy, as stress is increased, the strain increases to a point (X) where the material undergoes a transformation from the austenite to the martensite phase. Thereafter, stress remains substantially constant while strain is increased, forming a constant stress plateau (P). The superelastic material is reversibly deformable. It returns to its original length on curve (Y) as the stress is released. This cycle may occur repeatedly, without appreciable change in dimension or plastic deformation.

In Fig. 4a, the schematic stress-strain curve for a superelastic precursor material having linear elastic properties is shown for comparison to Fig. 4. The strain in this case increases reversibly to the plastic yield point (Z) without passing through a substantial plateau. At and above the yield point, the material becomes plastically, irreversibly deformed. In the present invention, at least the taper 16 or distal end 18 of the core member 12 is a linear elastic material formed of the precursor alloy of a superelastic material.

A preferred precursor of a superelastic alloy is a nickel-titanium (55%/45%) system (available as Alloy Bu from Ray-Chem Corp., Menlo Park, CA). Precursors of other superelastic alloys may also be used. These include e.g., Silver-Cadmium (Ag-Cd), Gold-Cadmium (Au-Cd), Gold-Copper-Zinc (Au-Cu-Zn), Copper-Aluminum-Nickel (Cu-Al-Ni), Copper-Gold-Zinc (Cu-Au-Zn), Copper-Zinc (Cu-Zn), Copper-Zinc-aluminum (Cu-Zn-Al), Copper-Zinc-Tin (Cu-Zn-Sn), Copper-Zinc-Xenon (Cu-Zn-Xe), Iron Beryllium (Fe₃Be), Iron-Platinum (Fe₃Pt), Indium-Thallium (In-Tl), iron-maganese (Fe-Mn) Nickel-Titanium-Vanadium (Ni-Ti-V), Iron-Nickel-Titanium-Cobalt (Fe-Ni-Ti-Co) and Copper-Tin (Cu-Sn). See Schetsky, L. McDonald, "Shape Memory Alloys", Encyclopedia of Chemical Technology (3rd ed.), John Wiley & Sons, 1982, vol. 20, pp. 726-736 for a full discussion of superelastic alloys.

Referring back to Figs. 1 to 3, in one embodiment for use in angiography, core element 12, is typically about 145 cm long or about 450cm long for a GI wire, has a body portion 14 of, e.g., about 0.5 to 7.4 mm (0.020 to 0.029 inch) diameter, and a tip portion 18, e.g., about 0.178 mm (0.007 inch) diameter that is about 2 to 10 cm long, with a smoothly tapering portion 16, e.g., about 7 to 10 cm long, all formed of a superelastic precursor alloy. The tapered portion of the wire 12 could also be continuous (not shown) rather than stepped as recited herein above. Body portion 14 of the core forms a generally stiffer region for torque transmission while the end section 18 and the tapering portion 16 at the distal end of the guidewire are of relatively greater flexibility due entirely to the reduced diameter. "GI" denotes gastrointestinal.

Referring now to Fig.2, the guidewire may include a polymer coating 22, e.g., polytetrafluoroethylene, Teflon (Trademark), disposed over the core 12 to facilitate smooth motion through body lumens. Other embodiments might include a lubricious coating as described, for example, by Terumo in EP-A-166998. An antithrombogenic coating may also be applied.

Referring now to Fig. 3, a spring coil member 19 may also extend over a portion, such as the distal tip, of the core. The spring coil aids in transmission of torque to the distal end when manipulating the proximal portion to navigate tortous lumens. Preferably, the coil is highly flexible, more flexible than the core, so that the coil may easily conform to the shape of the core when curvature is imparted by the physician. A radiopaque platinum coil is also preferable since it can facilitate observing and positioning of the element using radioscopic means.

It will also be understood that the precursor material could be used for just the tip or taper of the guidewire.

### Use

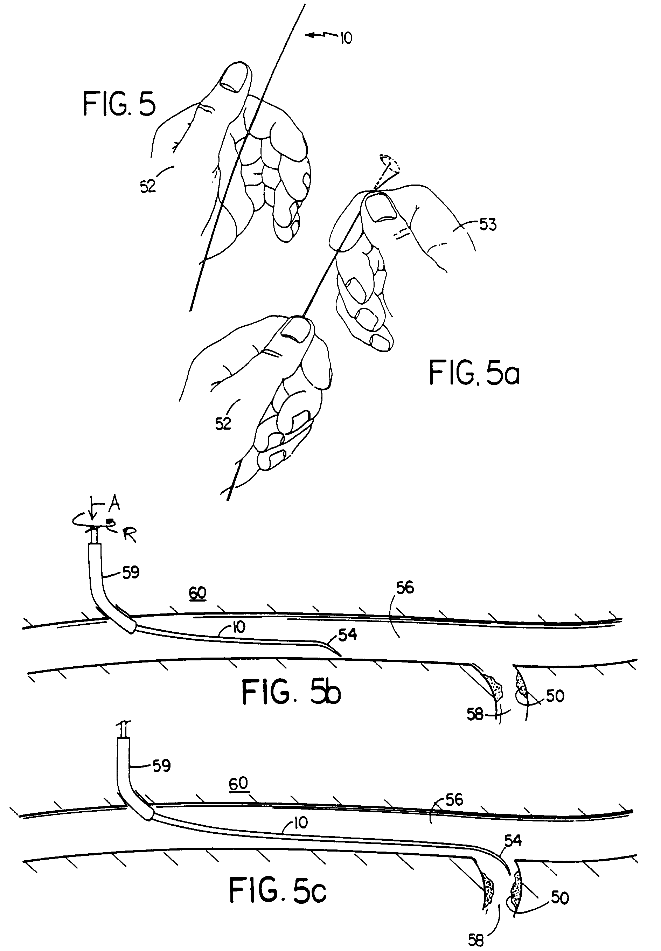

Referring to Figs. 5-5c, guidewire 10 can be used, for example, for treatment of vascular ailments. Generally, a physician inserts the distal end of guidewire 10 into a body lumen 56 such as a blood vessel. Just prior to entry into the body for locating the tip at, for example, the point of an occlusion 50 (Figs. 5b-5c), the distal tip may be preformed manually by the physician (figs. 5-5a) according to his judgement as to the degree and nature of curvature best for the situation. In Figs. 5-5a, the guidewire 10, may be taken from sterile storage and the physician, using his hands 52, 53, can grasp the end and shape the end to have the desired curvature 54 that complements the curvature of a lumen.

The guidewire 10 for angioplasty is inserted using, for example, the Seldinger technique, through an introducer sheath 59, placed in a limb 60, like the leg, to give access to the lumen 56 such as the femoral artery. Axial thrust (arrow A) is applied to the proximal portion to advance the guidewire 10 (Fig. 5b) in the lumen. In the example illustrated in the Figs. 5-5c, the curved end 54 of guidewire 10 is advanced through e.g, a large body lumen 56 to the desired position near a vessel 58, and steered by rotating body portion 14 (arrow R) to direct curved end 54 to the vessel 58 (Fig. 5c). (A G.I. guidewire may be introduced through an endoscope, (not shown), for gastrointestinal procedures, with benefits similar to the above-identified angioplasty procedure). A catheter can then be advanced over guidewire 10, and the guidewire removed when catheter is in place. Because of the high flexibility and elongation of the precursor of a superelastic alloy, the guidewire can negotiate highly tortuous passageways. Because the material is linearly elastic and deformable, the guidewire can be deformed by the physician just prior to entry for optimum shaping for ease of steering the element into a desired curved lumen.

### Manufacture

The guidewires of the present invention are formed of a precursor of a superelastic alloy. Guidewires that are substantially straight and capable of high elastic strain are manufactured from raw superelastic precursor element by taking care to prevent heating of the precursor that would result in heat treatment. In the event that excessive heating occurs, when the material is subsequently cooled, the straightness of the element may be lost. It also appears that in some cases the element becomes limp and less springy.

The precursor alloys, when first cold drawn from ingots into raw elements of constant diameter occur in the martensite phase at room temperature (as opposed to the superelastic alloy themselves which are drawn while heating and occur in the austenite phase at room temperature). The springiness and straightness in the martensite precursors we believe is a result of the cold drawing process which gives the martensite crystalline structure a preferred orientation. The high strain and straightness of the material can be maintained as long as the material is not substantially heated. When excessively heated, the materials pass through a transition to the austenite phase and, after cooling and returning to the martensite phase, the crystalline orientation or work hardening of the martensite which was imparted during the initial cold drawing is lost, leading to a less straight, and sometimes somewhat less spring material.

The temperature of the raw precursor element is kept below, preferably well below, (e.g., room temperature,) the transition temperature from martensite to austenite during processing of the precursor material when forming guidewires. For the Ni-Ti alloy (Alloy Bu) of the preferred embodiment, the transition temperature is approximately 149° C (300° F).

For forming a tapered guidewire, a tapering machine which renders a tapered section by center-less grinding with a friction wheel may be used. Such apparatus are available, for example, from Royal Master Corp, Oakland, New Jersey (models TG-12x3 (stepless) or TG-12x4). The friction of the grinding wheel against the alloy can lead to a rise of temperature of the material. To counter this, the rotation of the wheel, and the volume of liquid coolant flowing about the wheel and tip must be controlled. For example, on a Royal Master model TG-12x4, the rotation is reduced from a typical speed of about 40-50 rpm to about 19 rpm. The flow of room temperature coolant is typically half the maximum permitted by the model TG-12x4. Under these conditions, the tip may be ground to a taper without significantly increasing the temperature. It will be understood that various operating conditions might be adjusted and controlled to maintain a low temperature during the tapering step. For example, the temperature of the coolant might be reduced or its flow increased.

Other tapering methods could also be used, and the temperature of the grinding region controlled. For example, it also is known to form tapers by chemical etching or by drawing on an end or both ends of a element.

### Test Examples

### Example 1:

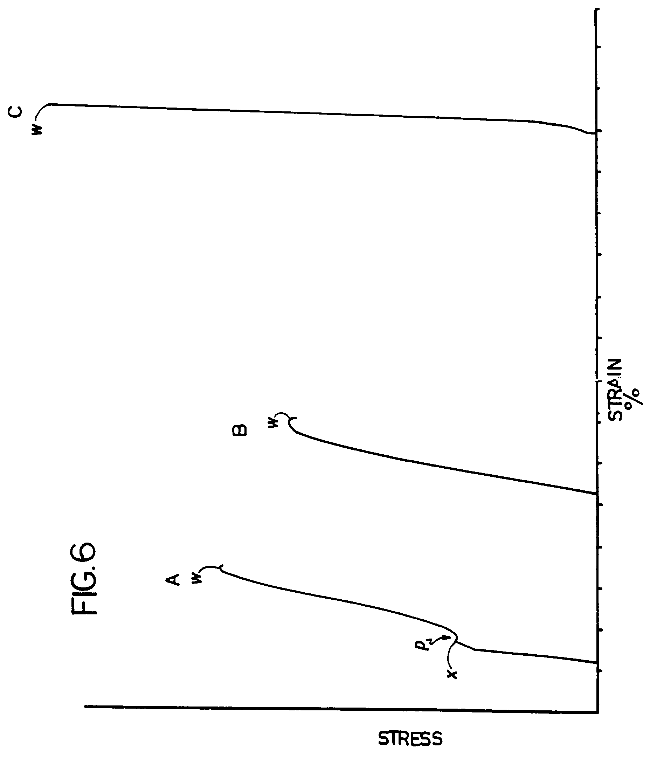

Referring now to Fig. 6, stress-strain curves from a stress testing machine for superelastic (curve A), superelastic precursor material (curve B) and a stainless steel element (curve C) are shown. The curves measure stress as a function of the percentage of strain for element pieces while drawing an end of the element. Stress testing machines are available from Instron Corp., Canton, MA. In Fig. 6, the percent strain is shown along the ordinate and measured stress along the abscissa.

In Fig. 6, curve A, the data shown is for a superelastic guidewire (Ni-Ti Alloy-BB, from Raychem Corp.) of about 0.5 mm (0.020 inch) outer diameter, Instron jaw separation 4 inches, speed 50.8 cm (20 inches)/min. The strain initially increases substantially linearly with stress up to point (x) at which point, the material transforms from austenite to martensite and stress is relatively constant with increasing strain, plateau (P). (The path y as in Fig. 4 is not shown since the Instron machine was operated to increase stress until the material failed by pulling apart at point (w)).

In Fig. 6, curve B, the Instron machine data shown is for a guidewire (0.5 mm) (0.020 inch) made from a linear elastic precursor (Alloy Bu) of the superelastic alloy tested in Fig. 6. In Fig. 6, curve C, the data for a stainless steel guidewire (0.5 mm) (0.020 inch) is shown. Both the linear elastic precursor and the stainless steel guidewires show no stress strain plateau indicating a phase change.

In the tests illustrated in Fig. 6, the strain before yield (plastic deformation) increased in order from stainless steel, to precursor, to superelastic.

### Example 2:

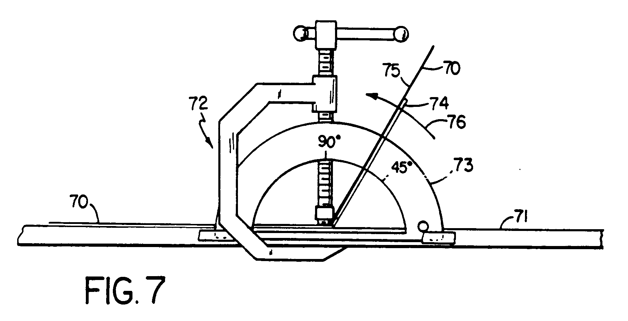

In table I, the bending angle at the yield point of a guidewire tip for stainless steel, a linear elastic precursor of a superelastic material and a superelastic material are given. Using the apparatus shown in Fig. 7, the elements were tested to determine the point of plastic deformation. A sample of element 70 was secured to a table 71 using a C-clamp 72. A protactor 73 was used to measure the angle. Using a steel ruler 74 a portion 75 of the element sample was moved in the direction of the arrow 76 until plastic deformation was observed. The diameter of the elements is given in mm (inches) in table I.

**Table I**

| Bending Angle at the Yield Point | | | |
|---|---|---|---|
| | Material | Diameter mm (inch) | Angle at Yield |
| Stainless Steel (Linear Elastic) | Stainless Steel | 0.5842 (0.0230) | 35° |
| Precursor of a Superelastic Alloy (Linear Elastic) | Alloy Bu | 0.5944 (0.0234) | 80° |
| Superelastic Alloy | Alloy BB | 0.5969 (0.0235) | 150° |

The stainless element easily takes a set and yields permanently at the 35° angle, whereas both the precursor of the superelastic and the superelastic were observed to take on only a very slight kink, taken to be an artifact of the test, at a first angle point (55° for precursor alloy and 60° for superelastic alloy BB). After this there occurred a broad range in arc (55-80° for the precursor; 60-150° for the superelastic) through which there was no additional change in permanent deformation until reaching the upper angle number shown in the table. At this point, significant permanent deformation is imparted to the elements.

## Claims

1. A guidewire device (10) having a distal portion comprised of an alloy having the composition of a superelastic alloy characterised in that said alloy has a linear stress-strain curve extending to a yield point without passing through a substantial plateau in which stress remains substantially constant while strain is varied, the distal alloy portion of said guidewire being deformable beyond said yield point to a desired set shape.

2. The device (10) of claim 1 wherein said alloy is selected from the group consisting of an alloy having a composition of In-Tl, Fe-Mn, Ni-Ti, Ag-Cd, Au-Cd, Au-Cu, Cu-Al-Ni, Cu-Au-Zn, Cu-Zn, Cu-Zn-Al, Cu-Zn-Sn, Cu-Zn-Xe, Fe₃Be, Fe₃Pt, Ni-Ti-V, Fe-Ni-Ti-Co, and Cu-Sn.

3. The device (10) of claim 2 wherein said alloy is selected from the group consisting of Ni-Ti, Cu-Al-Ni and Cu-Zn-Al.

4. The device (10) of claim 1 wherein said alloy is an alloy of about 55%/45% Ni-Ti.

5. The guidewire device (10) of any of the above claims in which at least part of said distal portion (16) is smaller than an integral portion of said guidewire proximal thereof, which is also comprised of said alloy.

6. The guidewire device (10) of claim 5 in which at least part of the guidewire formed of said alloy is tapered (16) from a larger to a smaller size in the direction of the distal end of said guidewire.

7. The guidewire device (10) of any of the above claims having a core element (14) comprised of said alloy, said core element extending proximally, integrally from said distal portion.

8. The device (10) of any of the above claims further comprising a polymeric external coating (22).

9. The device (10) of claim 8 wherein said coating (22) is polytetrafluoroethylene.

10. The device (10) of claim 8 wherein said coating (22) is a lubricous polymer.

11. The device (10) of claim 8 or 10 wherein said coating (22) includes an antithrombogenic agent.

12. The device (10) of claim 12 wherein said agent is heparin.

13. The device (10) of any of the above claims wherein said distal portion comprises an element (18) comprised of said alloy and further including a flexible spring coil (19) surrounding said element.

14. The device (10) of claim 13 wherein said spring coil (19) is more flexible than said element (18).

15. The device (10) of claim 6 wherein there is a portion (18) of said guidewire distal to said taper (16), said portion having diameter no greater than the diameter of the most distal portion of said taper.

16. The device (10) of claim 15 in the form of an angiographic guidewire having a total length of about 145 cm, a body portion (14) of about 0.5 to 7.4 mm (0.020 to 0.290 inch) diameter, a taper portion (16) about 7 to 10 cm long and a tip portion (18) about 2 to 10 cm long with a diameter of about 0.178 (0.007 inch), said body portion (14), taper portion (16) and tip portion (18) comprised of said alloy.

17. The device (10) of claim 15 in the form of a gastrointestinal guidewire having a total length of about 450 cm, a body portion (14) of about 0.5 to 7.4 mm (0.020 to 0.290 inch) diameter, a taper portion (16) about 7 to 10 cm long and a tip portion (18) about 2 to 10 cm long with a diameter of about 0.178 mm (0.007 inch), said body portion (14), taper portion (16) and tip portion (18) comprised of said alloy.

18. The device (10) of claim 6 wherein the distal portion of said guidewire continuously tapers distally to the smallest diameter at the distal end of the guidewire.

19. A method of forming a guidewire device from:
an element comprised of an alloy having the composition of a superelastic alloy, characterised in that said alloy has a linear stress-strain curve extending to a yield point, without passing through a substantial plateau in which stress remains substantially constant while strain is varied;
that the diameter of at least a portion of said element is reduced under conditions avoiding substantial heating;
and that, thereafter, the element is incorporated into a guidewire device, with said reduced diameter portion forming part of the distal portion of said guidewire device, said distal portion being elastically flexible while being capable of being given a desired permanent set by manual stressing of the distal region beyond its yield point.

20. The method of claim 19 wherein said element has a composition selected from In-Tl, Fe-Mn, Ni-Ti, Ag-Cd, Au-Cd, Au-Cu, Cu-Al-Ni, Cu-Au-Zn, Cu-Zn, Cu-Zn-Al, Cu-Zn-Sn, Cu-Zn-Xe, Fe₃Be, Fe₃Pt, Ni-Ti-V, Fe-Ni-Ti-Co, and Cu-Sn.

21. The method of claim 20 wherein said element is 55%/45% Ni-Ti.

22. The method of any of claims 19 to 21 including selecting an element in the martensite phase at room temperature to body temperature and maintaining said martensite phase during the formation of said guidewire.

23. The method of claim 22 wherein said maintaining includes maintaining the temperature of said element below the transition temperature to the austenite phase to avoid imparting superelasticity to said alloy.

24. The method of claim 23 wherein said element is maintained at room temperature.

25. The method of claim 22 including grinding at least part of said element to a reduced diameter while flooding said element with cooling fluid to control the temperature of said element.

26. The method of claim 25 further including controlling said grinding speed to control the temperature of said element.

27. The method of claim 25 or 26 wherein said temperature is below 149°C (300°F).

28. The method of claim 27 wherein said temperature is room temperature.

29. The method of claim 27 including grinding said element to provide a taper.

30. The method of any of the claims 19 to 21 further including drawing said element to produce said reduced diameter portion.

31. The method of any of the claims 19 to 21 further including chemical etching of said element to produce said reduced diameter portion.

32. The method of any of the above method claims further including applying to said element, a polymer coating.

33. The method of any of the above method claims further including applying to said element, a lubricous coating.

34. The method of any of the above method claims further including applying to said element, an antithrombogenic coating.

35. The method of claim 19 further including forming said element by cold drawing to impart said initial properties of elasticity and straightness.

36. The method of claim 19 wherein said device has a distal portion comprised of an alloy having a composition selected from the group consisting of an alloy of In-Tl, Fe-Mn, Ni-Ti, Ag-Cd, Au-Cd, Au-Cu, Cu-Al-Ni, Cu-Au-Zn, Cu-Zn, Cu-Zn-Al, Cu-Zn-Sn, Cu-Zn-Xe, Fe₃Be, Fe₃Pt, Ni-Ti-V, Fe-Ni-Ti-Co, and Cu-Sn, and at least part of the guidewire is tapered from a larger to a smaller size in the direction of the distal end of said guidewire where the tip of said guidewire is no larger than the most distal portion of said taper.

37. The method of claim 36 wherein there is a portion of said guidewire distal to said taper, said portion having diameter no greater than the diameter of the most distal portion of said taper.

38. The method of claim 36 wherein the distal portion of said guidewire continuously tapers distally to the smallest diameter at the distal end of the guidewire.

39. The method of any one of claims 36 to 38 wherein said alloy has a composition of about 55%/45% Ni-Ti.

40. The method of any of claims 19 to 39 wherein said alloy is in the martensite phase.

41. The method of claim 40 in which at least part of the guidewire formed of said alloy is tapered from a larger to a smaller size in the direction of the distal end of said guidewire.

42. The method of claim 40 or 41 wherein said alloy is about 55%/45% Ni-Ti.

## Patentansprüche

1. Führungsdraht-Vorrichtung (10) mit einem vorderen Abschnitt, der aus einer Legierung besteht, die die Zusammensetzung einer superelastischen Legierung hat, dadurch gekennzeichnet, daß die Legierung eine lineare Zug-Dehnungs-Kurve hat, die bis zur Streckgrenze geht, ohne daß sie durch einen im wesentlichen waagerechten Abschnitt verläuft, in dein der Zug im wesentlichen konstant bleibt, während sich die Dehnung ändert, wobei der vordere Legierungsabschnitt des Führungsdrahtes über die Streckgrenze zu einer gewünschten vorgegebenen Form verformbar ist.

2. Vorrichtung (10) nach Anspruch 1, wobei die Legierung aus der Gruppe ausgewählt ist, die aus einer Legierung besteht, die die Zusammensetzung In-Tl, Fe-Mn, Ni-Ti, Ag-Cd, Au-Cd, Au-Cu, Cu-Al-Ni, Cu-Au-Zn, Cu-Zn, Cu-Zn-Al, Cu-Zn-Sn, Cu-Zn-Xe, Fe₃Be, Fe₃Pt, Ni-Ti-V, Fe-Ni-Ti-Co und Cu-Sn hat.

3. Vorrichtung (10) nach Anspruch 2, wobei die Legierung aus der Gruppe ausgewählt ist, die aus Ni-Ti, Cu-Al-Ni und Cu-Zn-Al besteht.

4. Vorrichtung (10) nach Anspruch 1, wobei die Legierung eine Legierung von etwa 55 %/45 % Ni-Ti ist.

5. Führungsdraht-Vorrichtung (10) nach einem der vorstehenden Ansprüche, wobei zumindest ein Teil des vorderen Abschnitts (16) kleiner als der aus einem Stück bestehende Abschnitt des Führungsdrahts in seiner Nähe ist, der ebenfalls aus der Legierung besteht.

6. Führungsdraht-Vorrichtung (10) nach Anspruch 5, wobei zumindest ein Teil des aus der Legierung erzeugten Führungsdrahts in Richtung des vorderen Endes des Führungsdrahts von einer größeren zu einer geringeren Größe konisch (16) ist.

7. Führungsdraht-Vorrichtung (10) nach einem der vorstehenden Ansprüche, die ein Kernelement (14) aufweist, das aus dieser Legierung besteht, wobei das Kernelement sich unmittelbar anschließend, einstückig vom vorderen Abschnitt verläuft.

8. Vorrichtung (10) nach einem der vorstehenden Ansprüche, die außerdem eine polymere Außenbeschichtung (22) aufweist.

9. Vorrichtung (10) nach Anspruch 8, wobei die Beschichtung (22) Polytetrafluorethylen ist.

10. Vorrichtung (10) nach Anspruch 8, wobei die Beschichtung (22) ein gleitendes Polymer ist.

11. Vorrichtung (10) nach Anspruch 8 oder 10, wobei die Beschichtung ein antithrombogenes Mittel einschließt.

12. Vorrichtung (10) nach Anspruch 12, wobei das Mittel Heparin ist.

13. Vorrichtung (10) nach einem der vorstehenden Ansprüche, wobei der vordere Abschnitt ein Element (18) umfaßt, das aus der Legierung besteht und außerdem eine das Element umgebende flexible Federwicklung (19) aufweist.

14. Vorrichtung (10) nach Anspruch 13, wobei die Federwicklung (19) flexibler als das Element (18) ist.

15. Vorrichtung (10) nach Anspruch 6, wobei es einen vom Konus (16) entfernten Abschnitt (18) des Führungsdrahts gibt, wobei dieser Abschnitt einen Durchmesser aufweist, der nicht größer als der Durchmesser des entferntesten Abschnitts des Konus ist.

16. Vorrichtung (10) nach Anspruch 15 in Form eines angiographischen Führungsdrahts mit einer Gesamtlänge von etwa 145 cm, einem Körperabschnitt (14) mit einem Durchmesser von etwa 0,5 bis 7,4 mm (0,020 bis 0,290 inch), einem Konusabschnitt (16) mit etwa 7 bis 10 cm Länge und einem Spitzenabschnitt (18) mit etwa 2 bis 10 cm Länge und einem Durchmesser von etwa 0,178 (0,007 inch), wobei der Körperabschnitt (14), der Konusabschnitt (16) und der Spitzenabschnitt (18) aus der Legierung bestehen.

17. Vorrichtung (10) nach Anspruch 15 in Form eines gastrointestinalen Führungsdrahts mit einer Gesamtlänge von etwa 450 cm, einem Körperabschnitt (14) mit einem Durchmesser von etwa 0,5 bis 7,4 mm (0,020 bis 0,290 inch), einem Konusabschnitt (16) mit etwa 7 bis 10 cm Länge und einem Spitzenabschnitt (18) mit etwa 2 bis 10 cm Länge und einem Durchmesser von etwa 0,178 mm (0,007 inch), wobei der Körperabschnitt (14), der Konusabschnitt (16) und der Spitzenabschnitt (18) aus der Legierung bestehen.

18. Vorrichtung (10) nach Anspruch 6, wobei der vordere Abschnitt des Führungsdrahts entfernt zum kleinsten Durchmesser am vorderen Ende des Führungsdrahts kontinuierlich konisch ist.

19. Verfahren zur Herstellung einer Führungsdraht-Vorrichtung aus:
einem Element, das aus einer Legierung mit der Zusammensetzung einer superelastischen Legierung besteht, dadurch gekennzeichnet, daß die Legierung eine lineare Zug-Dehnungs-Kurve aufweist, die bis zur Streckgrenze geht, ohne daß sie durch einen im wesentlichen waagerechten Abschnitt verläuft, in dem der Zug im wesentlichen konstant bleibt, während sich die Dehnung ändert;
daß der Durchmesser von zumindest einem Abschnitt des Elementes bei Bedingungen verringert wird, die ein wesentliches Erwärmen vermeiden,
und daß das Element danach in eine Führungsdraht-Vorrichtung eingeführt wird, wobei der Abschnitt mit geringerem Durchmesser einen Teil des vorderen Abschnittes der Führungsdraht-Vorrichtung bildet, wobei der vordere Abschnitt elastisch flexibel ist, wobei er durch manuellen Zug des vorderen Bereichs über dessen Streckgrenze eine gewünschte bleibende Verformung erhalten kann.

20. Verfahren nach Anspruch 19, wobei das Element eine Zusammensetzung hat, die aus In-Tl, Fe-Mn, Ni-Ti, Ag-Cd, Au-Cd, Au-Cu, Cu-Al-Ni, Cu-Au-Zn, Cu-Zn, Cu-Zn-Al, Cu-Zn-Sn, Cu-Zn-Xe, Fe₃Be, Fe₃Pt, Ni-Ti-V, Fe-Ni-Ti-Co und Cu-Sn ausgewählt ist.

21. Verfahren nach Anspruch 20, wobei das Element 55 %/45 % Ni-Ti ist.

22. Verfahren nach einem der Ansprüche 19 bis 21, das das Auswählen eines Elements in der Martensit-Phase bei Raumtemperatur bis zur Körpertemperatur und das Halten dieser Martensit-Phase bei der Erzeugung des Führungsdrahts umfaßt.

23. Verfahren nach Anspruch 22, wobei das Halten das Halten der Temperatur des Elementes unterhalb der Übergangstemperatur in die Austenit-Phase umfaßt, damit vermieden wird, daß die Legierung Superelastizität erhält.

24. Verfahren nach Anspruch 23, wobei das Element bei Raumtemperatur gehalten wird.

25. Verfahren nach Anspruch 22, das das Schleifen von mindestens einem Teil des Elementes umfaßt, damit der Durchmesser verringert wird, während das Element mit einem Kühlfluid berieselt wird, damit die Temperatur des Elementes geregelt wird.

26. Verfahren nach Anspruch 25, das außerdem die Steuerung der Schleifgeschwindigkeit umfaßt, damit die Temperatur des Elementes geregelt wird.

27. Verfahren nach Anspruch 25 oder 26, wobei die Temperatur unter 149°C (300°F) liegt.

28. Verfahren nach Anspruch 27, wobei die Temperatur Raumtemperatur ist.

29. Verfahren nach Anspruch 27, das das Schleifen des Elementes umfaßt, damit ein Konus entsteht.

30. Verfahren nach einem der Ansprüche 19 bis 21, das außerdem das Strecken des Elementes umfaßt, damit der Abschnitt mit geringerem Durchmesser entsteht.

31. Verfahren nach einem der Ansprüche 19 bis 21, das außerdem das chemische Ätzen des Elementes umfaßt, wodurch der Abschnitt mit geringerem Durchmesser entsteht.

32. Verfahren nach einem der vorstehenden Verfahrensansprüche, das außerdem das Aufbringen einer Polymerbeschichtung auf das Element umfaßt.

33. Verfahren nach einem der vorstehenden Verfahrensansprüche, das außerdem das Aufbringen einer gleitfähigen Beschichtung auf das Element umfaßt.

34. Verfahren nach einem der vorstehenden Verfahrensansprüche, das außerdem das Aufbringen einer antithrombogenen Beschichtung auf das Element umfaßt.

35. Verfahren nach Anspruch 19, das außerdem das Formen des Elementes durch Kaltziehen umfaßt, wodurch die Anfangseigenschaften der Elastizität und Geradheit verliehen werden.

36. Verfahren nach Anspruch 19, wobei die Vorrichtung einen vorderen Abschnitt aufweist, der aus einer Legierung besteht, die eine Zusammensetzung aufweist, die aus der Gruppe ausgewählt ist, die aus einer Legierung von In-Tl, Fe-Mn, Ni-Ti, Ag-Cd, Au-Cd, Au-Cu, Cu-Al-Ni, Cu-Au-Zn, Cu-Zn, Cu-Zn-Al, Cu-Zn-Sn, Cu-Zn-Xe, Fe₃Be, Fe₃Pt, Ni-Ti-V, Fe-Ni-Ti-Co und Cu-Sn besteht, und mindestens ein Teil des Führungsdrahtes in Richtung des vorderen Endes des Führungsdrahtes von einer größeren zu einer geringeren Größe konisch ist, wobei die Spitze des Führungsdrahtes nicht größer als der entfernteste Abschnitt des Konus ist.

37. Verfahren nach Anspruch 36, wobei es einen vom Konus entfernten Abschnitt des Führungsdrahtes gibt, wobei dieser Abschnitt einen Durchmesser aufweist, der nicht größer als der Durchmesser des entferntesten Abschnitts des Konus ist.

38. Verfahren nach Anspruch 36, wobei der vordere Abschnitt des Führungsdrahtes entfernt vom kleinsten Durchmesser am vorderen Ende des Führungsdrahtes kontinuierlich konisch ist.

39. Verfahren nach einem der Ansprüche 36 bis 38, wobei die Legierung eine Zusammensetzung von etwa 55 %/45 % Ni-Ti hat.

40. Verfahren nach einem der Ansprüche 19 bis 39, wobei die Legierung in der Martensit-Phase ist.

41. Verfahren nach Anspruch 40, wobei zumindest ein Teil des aus der Legierung geformten Führungsdrahts in Richtung des vorderen Endes des Führungsdrahts von einer größeren zu einer geringeren Größe konisch ist.

42. Verfahren nach Anspruch 40 oder 41, wobei die Legierung etwa 55 %/45 % Ni-Ti ist.

## Revendications

1. Dispositif de guide (10) ayant une portion distale contenant un alliage ayant la composition d'un alliage superélastique, caractérisé en ce que ledit alliage a une courbe contrainte-déformation linéaire s'étendant jusqu'a une limite d'écoulement sans passer par un pallier substantiel où la contrainte reste sensiblement constante alors que la déformation varie, la portion distale en alliage dudit guide étant déformable au-delà de ladite limite d'écoulement jusqu'à une forme de réglage désirée.

2. Dispositif (10) selon la revendication 1, dans lequel ledit alliage est choisi dans le groupe comprenant les alliages de composition In-Tl, Fe-Mn, Ni-Ti, Ag-Cd, Au-Cd, Au-Cu, Cu-Al-Ni, Cu-Au-Zn, Cu-Zn, Cu-Zn-Al, Cu-Zn-Sn, Cu-Zn-Xe, Fe₃Be, Fe₃Pt, Ni-Ti-V, Fe-Ni-Ti-Co et Cu-Sn.

3. Dispositif (10) selon la revendication 2, dans lequel ledit alliage est choisi dans le groupe comprenant les alliages de composition Ni-Ti, Cu-Al-Ni et Cu-Zn-Al.

4. Dispositif (10) selon la revendication 1, dans lequel ledit alliage est un alliage de composition d'environ 55%/45% de Ni-Ti.

5. Dispositif (10) de guide selon l'une quelconque des revendications précédentes, dans lequel au moins une partie de ladite portion distale (16) est plus petite qu'une portion venue de matière dudit guide proximale de celle-ci, qui contient également ledit alliage.

6. Dispositif (10) de guide selon la revendication 5, dans lequel au moins une partie (16) du guide formée dudit alliage est rétrécie depuis une plus grande dimension jusqu'à une plus petite dimension dans la direction de l'extrémité distale dudit guide.

7. Dispositif (10) de guide selon l'une quelconque des revendications précédentes, ayant en outre un organe formant noyau (14) contenant ledit alliage, ledit organe formant noyau s'étendant de manière proximale et étant venu de matière depuis ladite portion distale.

8. Dispositif (10) selon l'une quelconque des revendications précédentes, comprenant en outre un revêtement externe polymérique (22).

9. Dispositif (10) selon la revendication 8, dans lequel ledit revêtement (22) est du polytetrafluoroethylène.

10. Dispositif (10) selon la revendication 8, dans lequel ledit revêtement (22) est un polymère de lubrification.

11. Dispositif (10) selon la revendication 8 ou 10, dans lequel ledit revêtement (22) inclut un agent antithrombogénique.

12. Dispositif (10) selon la revendication 11, dans lequel ledit agent est de l'héparine.

13. Dispositif (10) selon l'une quelconque des revendications précédentes, dans lequel ladite portion distale comprend un organe (18) contenant ledit alliage et comprenant en outre une bobine élastique souple (19) entourant ledit organe.

14. Dispositif (10) selon la revendication 13, dans lequel ladite bobine élastique (19) est plus souple que ledit organe (18).

15. Dispositif (10) selon la revendication 6, dans lequel il y a une portion (18) dudit guide distale par rapport audit rétrécissement (16), ladite portion ayant un diamètre non supérieur au diamètre de la portion la plus distale dudit rétrécissement.

16. Dispositif (10) selon la revendication 15 sous la forme d'un guide d'angiographie ayant une longueur totale d'environ 145 cm, une portion de corps (14) d'environ 0,5 à 7,4 mm (0,020 à 0,290 pouces) de diamètre, une portion de rétrécissement (16) d'environ 7 à 10 cm de long et une portion formant pointe (18) d'environ 2 à 10 cm de long avec un diamètre d'environ 0,178 mm (0,007 pouces), lesdites portion de corps (14) , portion de rétrécissement (16) et portion formant pointe (18) contenant ledit alliage.

17. Dispositif (10) selon la revendication 15, sous la forme d'un guide gastrointestinal ayant une longueur totale d'environ 450 cm, une portion de corps (14) d'environ 0,5 à 7,4 mm (0,020 à 0,290 pouces) de diamètre, une portion de rétrécissement (16) d'environ 7 à 10 cm de long et une portion formant pointe (18) d' environ 2 à 10 cm de long avec un diamètre d'environ 0,178 mm (0,007 pouces), les dites portion de corps (14), portion de rétrécissement (16) et portion formant pointe (18) contenant ledit alliage.

18. Dispositif (10) selon la revendication 6, dans lequel la portion distale dudit guide se rétrécit continûment de manière distale jusqu'au plus petit diamètre à l'extrémité distale du guide.

19. Procédé pour former un dispositif de guide à partir de :
un organe contenant un alliage ayant la composition d'un alliage superélastique, caractérisé en ce que ledit alliage a une courbe contrainte-déformation linéaire s'étendant jusqu'a une limite d'écoulement, sans passer par un pallier substantiel où la contrainte reste sensiblement constante alors que la déformation varie,
en ce que le diamètre d'au moins une portion dudit organe est réduite dans des conditions évitant un chauffage substantiel,
et en ce que, après cela, l'organe est incorporé dans un dispositif de guide, avec ladite portion de diamètre réduit formant partie de la portion distale dudit dispositif de guide, ladite portion distale étant souple de manière élastique tandis qu'on peut lui donner un réglage permanent souhaité par déformation manuelle de la région distale au-delà de sa limite d'écoulement.

20. Procédé selon la revendication 19, dans lequel ledit organe a une composition sélectionnée parmi les alliages de composition In-Tl, Fe-Mn, Ni-Ti, Ag-Cd, Au-Cd, Au-Cu, Cu-Al-Ni, Cu-Au-Zn, Cu-Zn, Cu-Zn-Al, Cu-Zn-Sn, Cu-Zn-Xe, Fe₃Be, Fe₃Pt, Ni-Ti-V, Fe-Ni-Ti-Co et Cu-Sn.

21. Procédé selon la revendication 20, dans lequel ledit organe a une composition de 55%/45% de Ni-Ti.

22. Procédé selon l'une quelconque des revendications 19 a 21, comprenant le choix d'un organe dans la phase martensitique à la température ambiante jusqu'à la température du corps et le maintien de ladite phase martensitique pendant la formation dudit guide.

23. Procédé selon la revendication 22, dans lequel ledit maintien comprend le maintien de la température de transition dudit organe sous la température vers la phase austénitique pour éviter de donner de la superélasticité audit alliage.

24. Procédé selon la revendication 23, dans lequel ledit organe est maintenu a la température ambiante.

25. Procédé selon la revendication 22, comprenant le meulage d'au moins une partie dudit organe jusqu'a un diamètre réduit tout en noyant ledit organe avec un fluide de refroidissement pour commander la température dudit organe.

26. Procédé selon la revendication 25, comprenant en outre la commande de la vitesse dudit meulage pour commander la température dudit organe.

27. Procédé selon la revendication 25 ou 26, dans lequel ladite température est sous 149°C (300°F).

28. Procédé selon la revendication 27, dans lequel ladite température est la température ambiante.

29. Procédé selon la revendication 27, comprenant le meulage dudit organe pour fournir un rétrécissement.

30. Procédé selon l'une quelconque des revendications 19 a 21, comprenant en outre l'étirage dudit élément pour produire ladite portion de diamètre réduit.

31. Procédé selon l'une quelconque des revendications 19 a 21, comprenant en outre l'attaque chimique dudit organe pour produire ladite portion de diamètre réduit.

32. Procédé selon l'une quelconque des revendications de procédé précédentes, comprenant en outre l'application audit organe d'un revêtement polymère.

33. Procédé selon l'une quelconque des revendications de procédé précédentes, comprenant en outre l'application audit organe d'un revêtement lubrifiant.

34. Procédé selon l'une quelconque des revendications de procédé précédentes, comprenant en outre l'application audit organe d'un revêtement antithrombogénique.

35. Procédé selon la revendication 19, comprenant en outre la mise en forme dudit organe par étirage à froid pour lui donner lesdites propriétés initiales d'élasticité et de rectitude.

36. Procédé selon la revendication 19, dans lequel ledit dispositif a une portion distale contenant un alliage ayant une composition choisie dans le groupe comprenant les alliages de composition In-Tl, Fe-Mn, Ni-Ti, Ag-Cd, Au-Cd, Au-Cu, Cu-Al-Ni, Cu-Au-Zn, Cu-Zn, Cu-Zn-Al, Cu-Zn-Sn, Cu-Zn-Xe, Fe₃Be, Fe₃Pt, Ni-Ti-V, Fe-Ni-Ti-Co et Cu-Sn, et au moins une partie du guide est rétrécie depuis une plus grande dimension jusqu'à une plus petite dimension dans la direction de l'extrémité distale dudit guide où la pointe dudit guide n'est pas plus grande que la portion la plus distale dudit rétrécissement.

37. Procédé selon la revendication 36, dans lequel il y a une portion dudit guide distale par rapport audit rétrécissement, ladite portion ayant un diamètre pas plus grand que le diamètre de la portion la plus distale dudit rétrécissement.

38. Procédé selon la revendication 36, dans lequel la portion distale dudit guide se rétrécit continûment distalement jusqu'au diamètre le plus petit au niveau de l'extrémité distale du guide.

39. Procédé selon l'une quelconque des revendications 36 à 38, dans lequel ledit alliage a une composition d'environ 55%/45% de Ni-Ti.

40. Procédé selon l'une quelconque des revendications 19 à 39, dans lequel ledit alliage est en phase martensitique.

41. Procédé selon la revendication 40, dans lequel au moins une partie du guide formé dudit alliage est rétrécie depuis une plus grande dimension jusqu'à une plus petite dimension dans la direction de l'extrémité distale dudit guide.

42. Procédé selon la revendication 40 ou 41, dans lequel ledit alliage est de composition d'environ 55%/45% de Ni-Ti.
